# EUROPEAN PATENT APPLICATION

(11) **EP 2 982 249 A1**
(43) Date of publication of application: **10.02.2016**
(21) Application number: 14192397.9
(22) Date of filing: 08.11.2014
(51) Int. Cl.: A23B 4/09

(54) **Freezer apparatus and method for treating a product**

(30) Priority: 04.08.2014 US 201414450570
(71) Applicant: Linde Aktiengesellschaft, 80331 München (DE)
(72) Inventor: Newman, Michael D., Hillsborough, NJ 08844 (US)
(74) Representative: Hofmann, Andreas

(57) **Abstract**

In order to overcome problems that earlier apparatus and methods have experienced, a freezer apparatus (10) for treating a product (14, 18), in particular a food product, with cryogen to kill bacteria on the product (14, 18) is proposed, comprising:
- a source of cryogen liquid, in particular comprising liquid nitrogen (LIN);
- a source of cryogen gas, in particular comprising gaseous nitrogen;
- a first conduit (42) for delivering a first amount of the cryogen liquid;
- a second conduit (58) in fluid communication with the first conduit (42) for delivering a second amount of the cryogen gas into the cryogen fluid for being mixed (61) therewith to provide a cryogen mixture having a ratio selected of cryogen gas to cryogen liquid; and
- a delivery apparatus in fluid communication with the first conduit (42) downstream of the second conduit (58), the delivery apparatus comprising at least one outlet (48) through which the cryogen mixture passes for contacting a surface of the product (14, 18).

A related method of treating a product (14, 18) is also proposed.

## Description

### Technical field of the present invention

The present invention relates to an apparatus and to a method for controlling heat flux at a surface of a product, for example of a food product, to kill campylobacter thereon.

### Technological background of the present invention

Campylobacter (meaning "twisted bacteria" due to its corkscrew appearance) is a bacteria recognized as one of the main causes of bacterial food borne disease in many developed countries. Transmission of the bacteria commonly occurs during ingestion of contaminated food or water, and the eating of raw meat. Campylobacter is frequently found in raw chicken products, although other food products have exhibited signs of these bacteria.

During freezing applications of, for example, food products in industrial settings, it is understood that a direct spray of a cryogen such as for example liquid nitrogen (LIN) onto the product will increase overall heat transfer to the product, i. e. the cooling rate of the product.

However, it is not known how to efficiently control the heat transfer rate of the product under spray nozzles which administer the liquid nitrogen, nor is there a known process that is economically beneficial to efficiently improve the heat transfer rates.

That is, known apparatus and methods do not permit an industrial plant to adjust or fine tune a heat transfer profile within a cryogenic freezing process of food or other products. In particular, and using a poultry carcass as an example, an area of surface treatment of the carcass with a cryogen such as liquid nitrogen requires a specific heat flux at the surface of the product to kill campylobacter.

In view of the cost of the cryogen used, if a heat flux is adjusted to be in excess of or not sufficient for the process, the resulting thermal shock will not be sufficient to kill the campylobacter at the surface of the carcass and will also result in an inefficient, wasteful and ineffective use of the cryogen.

### Disclosure of the present invention: object, solution, advantages

Taking the prior art as discussed into account, an object of the present invention is to overcome problems that earlier apparatus and methods have experienced. This object is accomplished by an apparatus comprising the features of claim 1 as well as by a method comprising the features of claim 12. Advantageous embodiments and expedient improvements of the present invention are disclosed in the respective dependent claims.

The present invention basically provides for an apparatus and for a method, wherein liquid nitrogen (LIN) sprays to be administered to products, in particular to food products and/or to animal carcasses, have a ratio of LIN gas to LIN liquid so that a consistent, uniform droplet size of the atomized LIN always be used to provide uniform heat transfer at the surface of the carcass and/or product.

In order to efficiently and optimally use LIN as the cryogen, the spraying of such is with same having a consistency of atomized particles of LIN in an amount sufficient to destroy the campylobacter, but not to waste the LIN or freeze the product. The apparatus according to the present invention and the method according to the present invention therefore provide the LIN in a spray with atomized particles being of just the right dimensions to both kill the campylobacter, but not waste the LIN or freeze the product.

The present invention provides an optimum droplet size of LIN in order to produce a heat transfer coefficient at a surface of the product, in particular of a specific food product, for example of a hamburger patty, or of the carcass, in order to destroy the campylobacter bacteria, such as for example destruction of same on a poultry carcass.

A resulting heat transfer at the surface of the carcass which produces a thermal shock in a temperature range which decreases at 600°C per minute provides the necessary application of destructive shock to this bacteria, without freezing the product or carcass.

There is therefore provided a freezer apparatus for treating a product, in particular for treating a food product or a carcass, with cryogen to kill bacteria on the product, which apparatus includes a source of cryogen liquid and a source of cryogen gas; a first conduit for delivering a first amount of the cryogen liquid; a second conduit in fluid communication with the first conduit for delivering a second amount of the cryogen gas into the cryogen fluid for being mixed therewith to provide a cryogen mixture having a ratio selected of cryogen gas to cryogen liquid; and a delivery apparatus in fluid communication with the first conduit downstream of the second conduit, the delivery apparatus including at least one outlet through which the cryogen mixture passes for contacting a surface of the product.

According to an advantageous embodiment of the present invention, at least one nozzle, in particular at least one spray nozzle, may be disposed at the at least one outlet through which the cryogen mixture passes. For providing at least one cryogen spray, the nozzle(s) may be arranged at a manifold or plenum, and the manifold or plenum may be arranged at a distal end of the first conduit.

According to an expedient embodiment of the present invention, a first valve, in particular a first control valve, for example operated on a separate proportional-integral-derivative (PID) loop, may be disposed for coaction with the first conduit for controlling flow of the cryogen liquid through the first conduit. More particularly, the first valve may be controlled based on liquid nitrogen (LIN) demand of a tunnel freezer and flow through the first conduit, with a temperature for the tunnel freezer process determining to what extent the first valve may be actuated to control the flow of the LIN to the tunnel freezer.

In connection therewith or independently thereof, a second valve may be disposed for coaction with the second conduit for controlling flow of the cryogen gas through the second conduit. More particularly, the second valve in the second conduit controls the amount of nitrogen gas being discharged into the liquid stream, i. e. into the first conduit. A portion of the cryogen mixture may be directed to the tunnel freezer and/or to a spray tunnel for use therewith.

According to a favoured embodiment of the present invention, a controller may be in communication with the second valve, said controller in particular operable to control actuation of said second valve with respect to the first amount of cryogen liquid. More particularly, the controller may monitor and regulate the output from the second valve, and optionally also from the first valve, so that the desired quality of gaseous fluid is reached upon entrance of the two phase flow into the cryogenic freezing system.

According to a preferred embodiment of the present invention, a flow meter may be disposed for metering the first amount of the cryogen liquid upstream of the second conduit. More particularly, the flow meter may be disposed for communication with the first conduit for metering or measuring the first amount of the cryogen liquid upstream of the second conduit.

According to an advantageous embodiment of the present invention, the controller may be in communication with each of the second valve and the flow meter, said controller in particular operable to control actuation of said second valve responsive to the cryogen liquid passing over said flow meter. More particularly, the controller may monitor and regulate the output from the second valve, and also from the first valve, so that the desired quality of gaseous fluid is reached upon entrance of the two phase flow into the cryogenic freezing system.

According to an expedient embodiment of the present invention, a spray tunnel may comprise a spray apparatus in fluid communication with the delivery apparatus and constructed to receive another product, in particular a carcass, for example a poultry carcass, such as a chicken carcass, in close proximity therewith, and a second outlet may be arranged for providing the cryogen mixture to said another product.

According to a favoured embodiment of the present invention, the second outlet may comprise at least another nozzle, in particular at least another spray nozzle, through which the cryogen mixture or cryogen spray passes, and for most applications a plurality of the nozzles to direct the cryogen mixture or cryogen spray onto said another product.

According to a preferred embodiment of the present invention, the spray apparatus may comprise a plurality of conduits, such as a pair of conduits, i. e. a third conduit and a fourth conduit spaced apart from said third conduit for providing a channel therebetween and through which said another product may pass. More particularly, each conduit may comprise at least one outlet, in particular at least one nozzle, for example at least one spray nozzle, and for most applications a plurality of the nozzles through which said cryogen mixture or cryogen spray may pass to contact the surface of said another product.

According to an advantageous embodiment of the present invention, a distal end of the second conduit may be in fluid communication with vapour, in particular with nitrogen vapour, present in a head space of the source of cryogen liquid for providing the source of cryogen gas. More particularly, the distal end of the second conduit may be connected to a head space of a nitrogen bulk storage tank or a vaporizer, depending on the required flow rates.

There is also provided a method of treating a product, in particular of treating a food product or a carcass, with cryogen to kill bacteria on the product, which method includes providing a source of cryogen liquid and a source of cryogen gas; providing a first amount of the cryogen liquid; providing a second amount of the cryogen gas into the cryogen liquid; mixing the first amount of the cryogen liquid and the second amount of the cryogen gas for providing a cryogen mixture having a ratio selected of cryogen gas to cryogen liquid; and applying said cryogen mixture to a surface of the product in an amount sufficient to destroy bacteria at said surface but not freeze the product.

According to an expedient embodiment of the present invention, the first amount and the second amount being mixed together may be controlled. More particularly, mixing of the fluid and of the gas results in the cryogen droplets sized to provide an effective and efficient heat transfer at the products, in particular at the food products and/or at the carcasses. Even more particularly, the consistency of the droplet size upon mixing does not change in any appreciable manner when the sprays are emitted from their respective nozzles.

According to a favoured embodiment of the present invention, a portion of the cryogen mixture may be directed to a remote location for other use, in particular to a spray tunnel for use therewith.

According to a preferred embodiment of the present invention, said portion may be split into at least two streams spaced apart and between which the product, in particular a carcass, for example a poultry carcass, such as a chicken carcass, passes in a channel for being contacted on at least two sides or surfaces by said at least two streams. More particularly, each stream may be directed to at least one outlet, in particular to at least one nozzle, for example at least one spray nozzle, and for most applications to a plurality of the nozzles through which said stream may pass to contact the side(s) or surface(s) of said product.

According to an advantageous embodiment of the present invention, the providing the second amount of cryogen gas may originate from vapour at the source of cryogen liquid. More particularly, a distal end of the second conduit may be in fluid communication with vapour, in particular with nitrogen vapour, present in a head space of the source of cryogen liquid for providing the source of cryogen gas. Even more particularly, the distal end of the second conduit may be connected to a head space of a nitrogen bulk storage tank or a vaporizer, depending on the required flow rates.

The cryogen mixture may be selected having a ratio of for example ten percent cryogen gas to ninety percent cryogen liquid to be used to kill the bacteria, while a cryogen mixture may be selected having a ratio of eighty percent cryogen gas to twenty percent cryogen liquid which is cost-effective for destroying bacterial such as campylobacter.

The cryogen gas can be gaseous nitrogen or may comprise gaseous nitrogen, and/or the cryogen liquid can be liquid nitrogen (LIN) or may comprise liquid nitrogen (LIN).

The present invention can be applied for heat flux control for liquid nitrogen (LIN) sprays, for example during carcass treatment applications for disinfection of the carcasses, such as with respect to killing campylobacter, and also during cryogenic tunnel freezing applications where high heat transfer coefficients are used.

### Brief description of the drawings

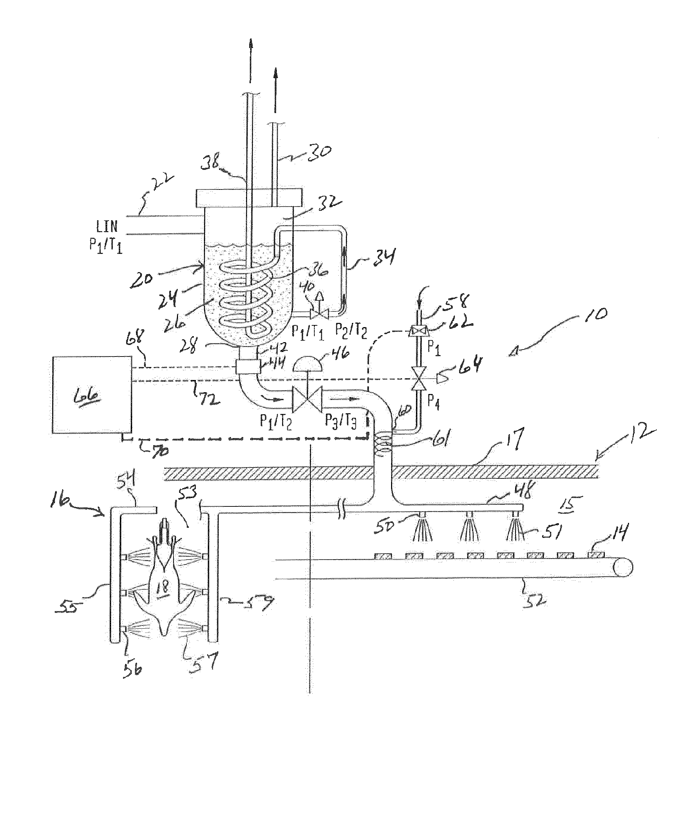

For a more complete understanding of the present embodiment disclosures and as already discussed above, there are several options to embody as well as to improve the teaching of the present invention in an advantageous manner. To this aim, reference may be made to the claims dependent on claim 1 as well as on claim 12; further improvements, features and advantages of the present invention are explained below in more detail with reference to particular and preferred embodiments by way of nonlimiting example and to the appended drawing figure taken in conjunction with the following description of the embodiments, of which:

The drawing figure shows a schematic view of an apparatus for also providing a method to treat a product, in particular a food product, with a cryogen to destroy campylobacter, according to the present embodiments.

### Detailed description of the drawings; best way of embodying the present invention

Referring to the drawing figure, an apparatus is shown generally at 10 which can be used with for example a tunnel freezer 12 for food products 14 and a spray tunnel 16 for providing a cryogenic spray for heat transfer/heat flux to implement a thermal shock to a carcass 18, for example to a poultry carcass, such as to a carcass of a chicken, or for providing controlled heat transfer to, for example hamburger patties or any other food product.

The apparatus 10 can be retrofit to existing freezer and tunnel assemblies and systems. The embodiment shown by way of example only can be used to provide a direct spray of liquid nitrogen (LIN) on poultry carcasses for treatment of campylobacter and similarly to provide liquid nitrogen sprays in the tunnel freezer 12 for application to the food product 14 as shown.

The quality of the LIN selected for use will have a direct affect on droplet size and spray distribution of the nitrogen being discharged for use in the freezer 12 and the tunnel 16. The "quality of the LIN" as used herein means the percentage of gas and the percentage of liquid that make up the fluid being introduced into the tunnel freezer 12 and the spray tunnel 16 to contact the food products 14 and the carcasses 18.

Accordingly, the apparatus 10 of the present embodiments can be adjusted and fine tuned until an optimum heat flux can be provided to both the food products 14 and/or the carcasses 18 to destroy campylobacter, without freezing the products and carcasses.

The apparatus 10 includes a phase separator 20 for receiving liquid nitrogen (LIN) from an inlet pipe 22 in communication therewith, the inlet pipe 22 in fluid communication with a bulk tank source (not shown) of the cryogen liquid. The tank may be a nitrogen bulk tank, by way of example only.

The phase separator 20 consists of a tank or vessel 24 to hold the cryogen liquid 26 (the LIN). A bottom of the vessel 24 is provided with an outlet 28 from which the liquid 26 can be removed from the vessel. A vent 30 is in communication with a gas space 32 present above a surface of the cryogenic liquid 26.

A stream of the cryogenic liquid 26 is drawn out of the vessel 24 through a side stream pipe 34. The pipe 34 extends upward to return the liquid 26 removed to an upper portion of the vessel 24, whereupon the pipe is coiled at 36 in a direction downward into the vessel 24 from where it again reverses direction and extends upward to exit from the vessel as shown at 38 in the drawing figure. The sidestream pipe 34 is provided with a control valve 40 to regulate an amount of the cryogen liquid 26 being removed from the vessel 24.

A pipe main 42 or main has a proximate end connected to the outlet 28 of the vessel 24. The main 42 is provided with a flow meter 44 and a control valve 46. The flow meter 44 is disposed for communication with the main 42 for metering or measuring a first amount of the cryogen liquid upstream of a gas inlet pipe 58 or conduit.

A distal end of the main 42 is provided with or connected to a manifold 48 or plenum which may have at least one spray nozzle 50 or perhaps a plurality of the nozzles 50 disposed for use within a chamber 15 of the tunnel freezer 12 to provide at least one cryogen spray 51 for the chamber 15. The main 42 extends through a sidewall 17 of the freezer 12. A conveyor apparatus 52, in particular a conveyor belt, transports the food products 14 through the chamber 15 of the tunnel freezer 12.

The manifold 48 may also be in fluid communication with a spray apparatus 54 of the spray tunnel 16. The spray apparatus 54 may also include at least one spray nozzle 56 and for most applications a plurality of the spray nozzles 56 to direct a cryogen spray 57 onto the carcass 18.

It should be understood that the tunnel freezer 12 and the spray tunnel 16 are shown by a way of example only with respect to their operable association with each other. That is, it is not necessary that the spray tunnel 16 is actually mounted to or formed integral with the tunnel freezer 12.

Rather, the distal end of the main 42 can be branched off to provide the manifold 48 and the spray apparatus 54 which can be constructed as separate assemblies in relatively close proximity to each other, but in different housings as required by the particular processing application.

The spray apparatus 54 may also be formed with a plurality of conduits, such as a pair of conduits 55, 59 spaced apart from each other for providing a channel 53 therebetween through which the product 18 (such as for example a food product) or carcass may pass. The conduits 55, 59 may each include at least one spray nozzle 56 for providing the cryogen spray 57.

The apparatus 10 also includes a gas inlet pipe 58 which has one end in fluid communication with a nitrogen gas tank (not shown) and an opposite or distal end in fluid communication with the main 42 as shown at 60 in the drawing figure. The gas from the gas tank flows through the pipe 58, which also includes a flow meter 62 and a control valve 64.

A controller 66 is in communication with the flow meter 44, the flow meter 62 and the control valve 64 as shown respectively by the broken lines 68, 70, 72 (collectively referred to as 68 to 72). The communication lines 68 to 72 can operate wirelessly if necessary.

Control valve 46 can be operated on a separate proportional-integral-derivative (PID) loop and is controlled based on liquid nitrogen (LIN) demand of the tunnel freezer 12 and flow through the main 42. A temperature for the tunnel freezer process will determine to what extent the valve 46 is actuated to control the flow of the LIN to the tunnel freezer 12.

In operation of the apparatus 10, and as shown in the drawing figure, liquid nitrogen (LIN) from the bulk storage tank (not shown) is fed at pressure P1, temperature T1 as a saturated liquid into the phase separator 20. Any vapour created in the inlet pipe 22 is vented to atmosphere through the phase separator vent 30.

A small side stream of liquid nitrogen is taken from the vessel 24 in pipe 34 and passed through the control valve 40 or cryogenic regulator to reduce P1 and T1 to a pressure P2 and a temperature T2, respectively. The side stream pipe 34 and control valve 40 function as a subcooler to provide the pressure P2 and the temperature T2. This now colder side stream 34 is returned to the vessel 24 where it passes through the heat exchanger coil 36 submerged in the liquid nitrogen 26.

The colder side stream 34 subcools the liquid nitrogen 26 to the pressure P1 and the temperature T2. The now pure subcooled liquid (all vapour removed) is passed through the main 42 across the flow meter 44 and through the control valve 46. The control valve 46 regulates the flow of liquid nitrogen into the process. Upon discharge from the control valve 46, the liquid nitrogen experiences a pressure drop to a pressure P3 and a temperature drop to a temperature T3.

At this point in the main 42 the nitrogen is still subcooled with no vapour. Also at this point in the main 42, a nitrogen vapour is introduced into the liquid stream through the gas inlet pipe 58. A distal end of the pipe 58 may be connected to a head space of the nitrogen bulk storage tank (not shown) or a vaporizer, depending on the required flow rates.

Such an arrangement provides for vapour in the head space of the bulk storage tank not to be wasted, but rather to be used as the cryogen vapour provided through the gas inlet pipe 58. The control valve 64 in the pipe 58 controls the amount of nitrogen gas being discharged into the liquid stream. A portion of the cryogen mixture may be directed to the tunnel freezer 12 and the spray tunnel 16 for use therewith.

The apparatus 10 permits an operator of same to control and regulate a droplet size of cryogen to be administered to the food products 14 and/or the carcasses 18. It is the droplet size of the cryogen which will control and provide an effective heat transfer rate of the food product and the carcass.

That is, in order to obtain the optimum size of the droplets emitted from the nozzles 50, 56, the nitrogen gas has to be injected into the liquid moving through the main 42 below the control valve 46 to provide the desired percentage ratio of gas to liquid.

The present apparatus 10 provides for a uniform and consistent droplet size of the cryogen being administered to the food product 14 and/or the carcass 18. It has therefore been determined herein that the quality of the LIN may be in a ratio of eighty percent gas to twenty percent liquid to effectively destroy the campylobacter bacteria but not freeze the product. This also provides for an efficient and cost-effective use of the LIN.

Both liquid and gaseous flow meters 44, 62, respectively, are installed in respective pipelines to monitor flow rates. The flow meter 44 is disposed for metering the cryogen liquid upstream of the gas inlet pipe 58. A desired liquid quality i. e. specific ratio of gas to liquid, can be entered into the controller 66.

The controller 66 monitors and regulates the output from the control valves 46, 64 so that the desired quality of gaseous fluid is reached upon entrance of the two phase flow into the cryogenic freezing system. Accordingly, the quality of the LIN downstream of where the gas inlet pipe 58 is in communication with the main 42 is shown generally at 61 where mixing of the fluid and gas results in the cryogen droplets sized to provide an effective and efficient heat transfer at the products 14 and the carcasses 18.

The consistency of the droplet size upon mixing 61 does not change in any appreciable manner when the sprays 51, 57 are emitted from their respective nozzles 50, 56.

An embodiment of the apparatus 10 can be used with direct spray of atomized liquid nitrogen onto the food products 14 and the poultry carcasses 18 for treatment of campylobacter. The quality of liquid has a direct effect on both droplet size and spray distribution of nitrogen being discharged from the nozzles 50, 56. The system can therefore be fine tuned until the optimum heat flux is reached for the process.

It will be understood that the embodiments described herein are merely exemplary, and that one skilled in the art may make variations and modifications without departing from the spirit and scope of the invention. All such variations and modifications are intended to be included within the scope of the invention as described and claimed herein. Further, all embodiments disclosed are not necessarily in the alternative, as various embodiments of the invention may be combined to provide the desired result.

### List of reference numerals

- 10: apparatus, in particular freezer apparatus
- 12: freezer, in particular tunnel freezer
- 14: product, in particular food product, for example hamburger patty
- 15: chamber of freezer 12
- 16: tunnel, in particular spray tunnel
- 17: sidewall of freezer 12
- 18: further or other product, in particular carcass, for example poultry carcass, such as chicken carcass
- 20: phase separator
- 22: inlet pipe
- 24: tank or vessel
- 26: cryogen(ic) liquid, in particular liquid nitrogen
- 28: outlet of tank or vessel 24
- 30: vent, in particular phase separator vent
- 32: gas space
- 34: pipe, in particular side stream pipe
- 36: coil, in particular heat exchanger coil
- 38: exit
- 40: control valve or cryogenic regulator
- 42: first conduit or first pipe, in particular main, for example pipe main
- 44: first flow meter, in particular liquid flow meter
- 46: first valve, in particular first control valve
- 48: outlet, in particular manifold or plenum
- 50: first nozzle, in particular first spray nozzle, for cryogen spray 51
- 51: cryogen spray for chamber 15
- 52: conveyor apparatus, in particular conveyor belt
- 53: channel between first conduit 55 and second conduit 59
- 54: spray apparatus of tunnel 16
- 55: first conduit of spray apparatus 54
- 56: second nozzle, in particular second spray nozzle, for cryogen spray 57
- 57: cryogen spray
- 58: second conduit or second pipe, in particular gas inlet conduit or gas inlet pipe
- 59: second conduit of spray apparatus 54
- 60: distal or opposite end of second conduit or second pipe 58 in fluid communication with first conduit or first pipe 42
- 61: mixing first amount of cryogen liquid and second amount of cryogen gas
- 62: second flow meter, in particular gaseous flow meter
- 64: second valve, in particular second control valve
- 66: controller
- 68: communication line, in particular wireless communication line, between first flow meter 44 and controller 66
- 70: communication line, in particular wireless communication line, between second flow meter 62 and controller 66
- 72: communication line, in particular wireless communication line, between second valve 64 and controller 66
- P1: first pressure
- P2: second pressure
- P3: third pressure
- T1: first temperature
- T2: second temperature
- T3: third temperature

## Claims

1. A freezer apparatus (10) for treating a product (14, 18), in particular a food product, with cryogen to kill bacteria on the product (14, 18), comprising:
- a source of cryogen liquid, in particular comprising liquid nitrogen (LIN);
- a source of cryogen gas, in particular comprising gaseous nitrogen;
- a first conduit (42) for delivering a first amount of the cryogen liquid;
- a second conduit (58) in fluid communication with the first conduit (42) for delivering a second amount of the cryogen gas into the cryogen fluid for being mixed (61) therewith to provide a cryogen mixture having a ratio selected of cryogen gas to cryogen liquid; and
- a delivery apparatus in fluid communication with the first conduit (42) downstream of the second conduit (58), the delivery apparatus comprising at least one outlet (48) through which the cryogen mixture passes for contacting a surface of the product (14, 18).

2. The freezer apparatus according to claim 1, further comprising at least one nozzle (50), in particular at least one spray nozzle, disposed at the at least one outlet (48) and through which the cryogen mixture passes.

3. The freezer apparatus according to claim 1 or 2, further comprising
- a first valve (46), in particular a first control valve, for example operated on a separate proportional-integral-derivative (PID) loop, disposed for coaction with the first conduit (42) for controlling flow of the cryogen liquid through the first conduit (42), and
- a second valve (64), in particular a second control valve, disposed for coaction with the second conduit (58) for controlling flow of the cryogen gas through the second conduit (58).

4. The freezer apparatus according to claim 3, further comprising a controller (66) in communication with the second valve (64), said controller (66) operable to control actuation of said second valve (64) with respect to the first amount of cryogen liquid.

5. The freezer apparatus according to at least one of claims 1 to 4, further comprising a flow meter (44) disposed for metering the first amount of the cryogen liquid upstream of the second conduit (58).

6. The freezer apparatus according to claim 5, further comprising a controller (66) in communication with each of the second valve (64) and the flow meter (44), said controller (66) operable to control actuation of said second valve (64) responsive to the cryogen liquid passing over said flow meter (44).

7. The freezer apparatus according to at least one of claims 1 to 6, further comprising a spray tunnel (16), said spray tunnel (16) comprising a spray apparatus (54) in fluid communication with the delivery apparatus and constructed to receive another product (18) in close proximity therewith, and a second outlet for providing the cryogen mixture to said another product (18).

8. The freezer apparatus according to claim 7, wherein the second outlet comprises at least another nozzle (56), in particular at least another spray nozzle, through which the cryogen mixture passes.

9. The freezer apparatus according to claim 7 or 8, wherein the spray apparatus (54) comprises a third conduit (55) and a fourth conduit (59) spaced apart from said third conduit (55) for providing a channel (53) therebetween and through which said another product (18) may pass, and at least a third outlet in said third conduit (55) and at least a fourth outlet in said fourth conduit (59) through which said cryogen mixture may pass to contact the surface of said another product (18).

10. The freezer apparatus according to at least one of claims 1 to 9, wherein a distal end of the second conduit (58) is in fluid communication with vapour present in a head space of the source of cryogen liquid for providing the source of cryogen gas.

11. The freezer apparatus according to at least one of claims 1 to 10, wherein the cryogen mixture comprises a ratio of cryogen gas between ten percent and eighty percent to cryogen liquid between ninety percent and twenty percent, in particular a ratio of eighty percent cryogen gas to twenty percent cryogen liquid.

12. A method of treating a product (14, 18), in particular of treating a food product, with cryogen to kill bacteria on the product (14, 18), comprising:
- providing a source of cryogen liquid, in particular comprising liquid nitrogen (LIN),
- providing a source of cryogen gas, in particular comprising gaseous nitrogen;
- providing a first amount of the cryogen liquid;
- providing a second amount of a cryogen gas into the cryogen liquid;
- mixing (61) the first amount of the cryogen liquid and the second amount of the cryogen gas for providing a cryogen mixture having a ratio selected of cryogen gas to cryogen liquid; and
- applying said cryogen mixture to a surface of the product (14, 18) in an amount sufficient for destroying bacteria at said surface but not freezing the product (14, 18).

13. The method according to claim 12,
- further comprising controlling the first amount and the second amount being mixed (61) together, and/or
- further comprising directing a portion of the cryogen mixture to a remote location for other use, in particular splitting said portion into at least two streams spaced apart and between which the product (18) passes for being contacted by said at last two streams.

14. The method according to claim 12 or 13, wherein the providing the second amount of cryogen gas originates from vapour at the source of cryogen liquid.

15. The method according to at least one of claims 12 to 14, wherein the cryogen mixture comprises a ratio of cryogen gas between ten percent and eighty percent to cryogen liquid between ninety percent and twenty percent, in particular a ratio of eighty percent cryogen gas to twenty percent cryogen liquid.
